# EUROPEAN PATENT SPECIFICATION

(11) **EP 0 498 243 B1**
(45) Date of publication and mention of the grant of the patent: **15.05.1996**
(21) Application number: 92101224.1
(22) Date of filing: 27.01.1992
(51) Int. Cl.: C07F 9/655, A61K 31/66, C07C 211/63

(54) **Fosfomycin cetyltrimethylammonium salt and composition containing it, for the topical treatment of vaginal and urethral infections**
Fosfomycin-Cetyltrimethylammonium-Salz und diese enthaltende Zusammensetzung für die lokale Behandlung von Vagina- und Harnröhre-Infektionen
Sel de cetyltrimethylammonium avec la phosphomycine et composition le contenant, pour le traitement local d'infections vaginales et urètrales

(30) Priority: 01.02.1991 IT MI910244
(43) Date of publication of application: 12.08.1992
(73) Proprietor: LA.FA.RE. S.r.l., I-80056 Ercolano (Napoli) (IT)
(72) Inventor: Marfe', Paolo, I-80040 San Sebastiano al Vesuvio, NA (IT); Di Schiena, Michele Giuseppe, I-20090 Cisliano, MI (IT)
(74) Representative: Giambrocono, Alfonso, Dr. Ing.

(56) References cited:
- GB-A- 2 013 682

## Description

The present invention relates to fosfomycin cetiltrimethylammonium and to the compositions containing it, for the topical treatment in the vaginal and urethral infections.

Fosfomycin is a well-known antibiotic of formula :

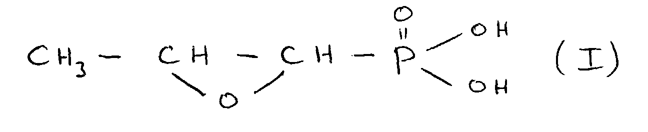

It has been used for the preparation of intravenous and parenterally administrable medicaments for the treatment of the diseases of the urinary apparatus, of the respiratory system, in surgical, affections, soft tissues and skin infections, in burns, in septicaemiae and in all Gram-positive and Gram-negative bacterial infections.

Some of the pharmaceutically acceptable salts of the compound of formula I are well known too and in particular sodium, calcium and trometamol salts are used for fosfomycin therapeutical applications. In the gynecological field, for instance, the fosfomycin sodium salt is used by intravenous injection or phleboclysis (Jpn. J. Antibiot. 1985, 38(8), 2045-56).

The administration of the sodium salt of fosfomycin may also reach significant and potentially dangerous amounts for women being hypertensive and cardiopathic or affected by metabolic diseases. In these patients the concentration of sodium ion in physiological fluids should be limited or possibly completely eliminated.

Furthermore, the administration of fosfomycin and of its salts in case of pregnancy and nursing requires much caution, as it can cause some systemic effects due to its powerful antimicrobial activity such as, for instance, intestinal dismicrobism, onset of resistant bacterial strains, hypersensitivity phenomena (gastro-intestinal disorders, rash).

The parenteral administration of fosfomycin and of its salts is also particularly painful for the patient.

For varius reasons, fosfomycin and its known salts are not used for the topical treatment of vaginal and urethral infections, because they are instable at room atmosphere, or because they are water-insoluble, or because they give esothermic reactions (therefore causing a great sense of burning on wounds and on mucous membranes on which they can be applied), or because their aqueous solutions are rather instable in time.

The fosfomycin cetyltrimethylammonium salt, is a compound stable at room atmosphere and to humidity (it crystallises also with 15% water) and can be easily handled and be constitutional part of different formulations, it does not give esothermic reaction when it comes into contact with humidity and therefore with skin wounds and with mucous membranes which are naturally humid (so it can be applied even as a powder); it is extremely water-soluble (and therefore it is suitable for a great number of pharmaceutical forms for vaginal use); and in the end its aqueous solutions are significantly stable in time.

It has been now surprisingly found that fosfomycin cetyltrimethylammonium salt and the compositions containing it, are particularly useful and effective for the treatment of vaginal and urethral infections such as for instance, acute and recurrent bacterial cystites, bacterial urethral and bladder syndromes, non specific bacterial urethrites, postoperative urinary infections; and furthermore in the prophylaxis of the urinary tract infections in surgical operations and in trans-urethral diagnostic handlings, before and after the IUD applications, in gynecological pre- and post-operative prophylaxis, in private hygiene during puerperium , in vulvovaginites and in exocervicites of bacterial origin including those deriving from chemo- and radio- therapy, in post-coital phrophylaxis of gonorrhoea, syphilis and other venereal infections.

The topical use of the fosfomycin cetyltrimethylammonium salt and of the preparations containing it as active principle, reduces and eliminates all the side effects caused by systemic administration, still allowing an effective therapy against pathogenic agents.

In particular, using fosfomycin cetyltrimethylammonium it is possible to topically perform an effective anti-bacterial therapy against cocci and bacilli and an effective anti-fungus therapy, in particular against the dangerous vaginal pathogenic agent Trichomonas vaginalis, and against moniliae (Candida albicans) which are usual presences in mucous membranes and which can also induce serious morbid forms (moniliasys or thrushes).

Furthermore it is important to notice that the fosfomycin cetyltrimethylammonium salt owns other peculiar properties, such as interesting surfactant and foaming properties particularly useful in making a washing, since its detergent action; when dissolved in water, it is also able to give a substantially neutral and therefore physiological solution even for vaginal mucous membrane which notoriously is greatly sensible; said salt therefore results particularly advisable in the vaginal infections therapy, avoiding also the onset of dangerous super-infections of particular microorganisms which are normally in equilibrium among themselves.

The compositions containing the fosfomycin cetyltrimethylammonium salt are prepared by well known methods and are useful for the preparation of pharmaceutical forms suitable for the topical vaginal and urethral use, such as, for instance, washings for vaginal and urethral irrigation, vaginal and urethral suppositories, globules, creams, vaginal tablets, powders for extemporary use, spray formulations, and the like.

The pharmaceutical carrier used for the preparation of said compositions is chosen among the ones known in the art and can vary depending on the pharmaceutical forms that one wants to prepare.

The amount of sodium fosfomycin used each time can also vary within wide ranges depending on the properties of the used formulation.

Generally speaking it can be stated that fosfomycin cetyltrimethylammonium can be used in an amount comprised between 0.1 g and 1 g and more particularly between 0.05 g and 2 g.

The following examples illustrate the invention without limiting it in any way.

### EXAMPLE 1

### Preparation of fosfomycin cetyltrimethylammonium

To a solution cooled at 0°C and maintained under stirring of 60.31 g (0.2 moles) of cetyl-trimethylammonium in 100 ml of methanol, an acid fosfomycin solution, 13.8 g in 1000 of methanol, is slowly added.

The resulting solution is left under stirring for 1 hour allowing the mixture temperature to rise to room temperature.

Evaporation under reduced pressure is effected at an external temperature of maximum 45°C.

The spongy residual is treated with 1000 ml of anhydrous acetone, stirring until complete transformation of the spongy solid into a crystalline solid. The solid is collected in vacuo, washed with acetone and air-dried or better under vacuum at a maximum temperature of 40°C.
Yield 98% of the theoretical weight.

White solid flakes having wax-like consistence.
NMR (solvent: CH₃OH: BRUKER AC 300): PPM 0.9 (m,3H), 1.2-1.4 (m), 1.7-1.9 (m), 1.57 (d, 3H), 2.81 (dd, 1H), 3.08 (m, 1H), 3.13 (s, 9H), 3.28-3.38 (m, 2H).

### EXAMPLE 2

Vaginal washing - ready gynecological solution, 100 ml contain:

| | |
|---|---|
| Fosfomycin cetyltrimethylammonium salt | 2.550 g |
| Salvoderm pink perfume | 0.100 g |
| Purified water q.s. to | 97.350 ml |

### EXAMPLE 3

Vaginal washing - monodose paper bag each bag contains:

| | |
|---|---|
| Fosfomycin cetyltrimethylammonium salt | 2.550 g |
| Sodium chloride | 8.800 g |
| Salvoderm pink perfume | 0.200 g |

### EXAMPLE 4

Multi - dose vial for vaginal and urethral irrigations, each vial contains:

| | |
|---|---|
| Fosfomycin cetyltrimethylammonium salt | 25.5 g |
| Asiatic "centella" glycolic extract | 10 g |
| Purified water q.s. to | 100 g |

## Claims (Claims for the following Contracting State(s): CH, DE, FR, GB, GR, IT, LI, PT)

1. Fosfomycin cetyltrimethylammonium salt.

2. Composition for the topical treatment of vaginal and urethral infections, characterized in that it comprises fosfomycin cetyltrimethylammonium salt and a pharmaceutically acceptable carrier.

## Claims (Claims for the following Contracting State(s): ES)

1. A process for preparing fosfomycin cetyl trimethylammonium which comprises contacting cetyl trimethylammonium with a fosfomycin solution, reco vering the resulting fosfomycin cetyltrimethyl ammonium salt according to known techniques.

2. A process for preparing a composition for the topical treatment of vaginal and urethral infe ctions, which comprises mixing the fosfomycin cetyl trimethylammonium salt with a pharmaceutically acce ptable carrier.

## Patentansprüche (Patentansprüche für folgende(n) Vertragsstaat(en): CH, DE, FR, GB, GR, IT, LI, PT)

1. Fosfomycin cetyltrimethylammoniumsalz.

2. Zusammemsetzung fur die topische Behandlung der vaginalen und urethralen Infektionen, dadurch gekennzeichnet dass sie Fosfomycin cetyltrimethylammoniumsalz und einen pharmazeutish zülassigen Träger umfasst.

## Patentansprüche (Patentansprüche für folgende(n) Vertragsstaat(en): ES)

1. Verfahren zur Herstellung des Fosfomycin cetyltrimethylammoniums, wobei das Verfahren die Reaktion des Cetyl trimethylammoniums mit einer Fosfomycinlosung, und die Verwertung des resultierenden Fosfomycin cetyltrimethylammoniumsalz gemäss der bekannten Techniken umfasst.

2. Verfahren zur Herstellung einer Zusammensetzung fur die topische Behandlung der vaginale und urethralen Infektionen, wobei das Verfahren die Mischung des Fosfomycin cetyltrimethylammoniumsalz mit einem pharmazeutish zulassigen Träger umfasst.

## Revendications (Revendications pour l'(les) Etat(s) contractant(s) suivant(s): CH, DE, FR, GB, GR, IT, LI, PT)

1. Sel de fosfomycin cetyltrimethylammonium.

2. Composition pour le traitement topique de infections vaginales et urétrales, caracterisèe en ce qu'elle comprend sel de fosfomycin cetyltrimethylammonium et un support pharmaceutiquement acceptable.

## Revendications (Revendications pour l'(les) Etat(s) contractant(s) suivant(s): ES)

1. Procédé pour la preparation de fosfomycin cetyl trimethylammonium, lequel procédé comprend la réaction de cetyl trimethylammonium avec une solution de fosfomycin, et le récupération du sel de fosfomycin cetyltrimethylammonium résultant selon les tecniques connues.

2. Procédé pour la preparation d'une composition pour le traitement topique de infections vaginales et urétrales, lequel procédé comprend la mélange du sel de fosfomycin cetyltrimethylammonium avec un support pharmaceutiquement acceptable
